(19) Europäisches Patentamt
European Patent Office
Office européen des brevets

(11) **EP 4 546 359 A2**

(12) **EUROPEAN PATENT APPLICATION**

(43) Date of publication:
**30.04.2025 Bulletin 2025/18**

(21) Application number: **24208159.4**

(22) Date of filing: **22.10.2024**

(51) International Patent Classification (IPC):
***G16H 20/17*** *(2018.01)* ***G16H 50/70*** *(2018.01)*

(52) Cooperative Patent Classification (CPC):
**G16H 20/17; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 US 202363592268 P**

(71) Applicant: **Insulet Corporation**
**Acton, MA 01720 (US)**

(72) Inventors:
- **LEE, Joon Bok**
**Acton, MA 01720 (US)**
- **ZADE, Ashutosh**
**San Diego, CA 92128 (US)**
- **ZHENG, Yibin**
**Hartland, WI 53029 (US)**
- **O'CONNOR, Jason**
**Acton, MA 01720 (US)**

(74) Representative: **Peterreins Schley**
**Patent- und Rechtsanwälte PartG mbB**
**Hermann-Sack-Straße 3**
**80331 München (DE)**

(54) **AUTOMATED MEDICAMENT DELIVERY SYSTEM MANAGEMENT WITHOUT ANALYTE MEASUREMENTS**

(57) A medicament delivery system, process and techniques are provided. The medicament delivery system may include a wearable medicament delivery device, a memory storing programming instructions, and a processor. The processor may be operable to execute the programming instructions, which cause the processor to receive an available medicament delivery history of a user, and determine whether the available medicament delivery history spans an extent of time greater than a first glucose history duration. The processor, in response to a determination that the extent of time spanned by the available medicament delivery history is greater than the first glucose history duration, determines an average daily medicament delivered. The processor calculates a trust value of the available medicament delivery history, and utilizes the trust value to determine a total daily medicament value of the user.

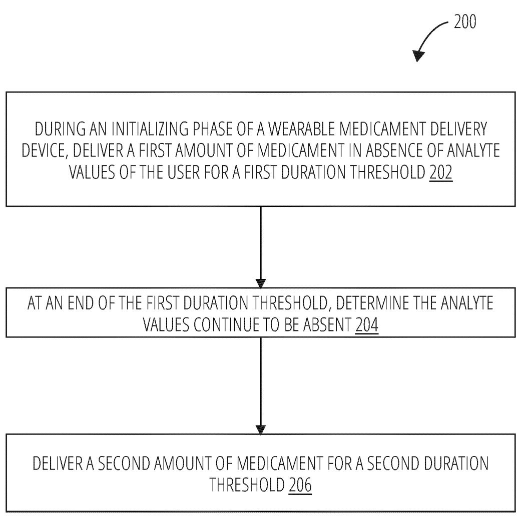


FIG. 2

EP 4 546 359 A2

## Description

### BACKGROUND

Automated medicament delivery (AMD) systems often distinguish between "automated mode" medicament delivery when glucose measurements are available, and "manual mode" medicament delivery when such measurements are not available. However, new users of such insulin pumps have difficulty understanding the differences between automated mode and manual mode. Next generation AMD systems attempt to eliminate this distinction, but face challenges in being able to deliver medicaments, such as insulin, without significant user input when glucose measurements are unavailable, particularly when there also is no history to estimate the user's insulin needs.

It would be beneficial if an AMD system was provided that was capable of initiating delivery of medicament accounting for unavailable glucose measurements or other measurements.

### BRIEF SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one aspect, a medicament delivery system is provided. The medicament delivery system may include a wearable medicament delivery device, a memory storing programming instructions, and a processor. The processor may be operable to execute the programming instructions, which cause the processor, during an initializing phase of the wearable medicament delivery device, to deliver a first amount of medicament in absence of analyte values of a user for a first duration threshold. The first amount of medicament is based on an assumption that the absent analyte levels are greater than a first glucose threshold. At an end of the first duration threshold, the processor determines whether the analyte values continue to be absent, and is operable to deliver a second amount of medicament for a second duration threshold based on the determination. The second amount of medicament is different from the first amount of medicament and the second amount of medicament is based on an assumption that the user's absent analyte levels are still greater than the first duration threshold.

In another aspect, another medicament delivery system is provided. The medicament delivery system may include a wearable medicament delivery device, a memory storing programming instructions, and a processor. The processor may be operable to execute the programming instructions, which cause the processor to receive a glucose history of a user having a first glucose history duration. A trust value is set by the processor based on the first glucose history duration. The processor calculates an estimated total daily medicament value utilizing the trust value. The processor receives an updated glucose history of the user having a second glucose history duration, and sets an updated trust value based on the second glucose history duration. The processor is operable to calculate a subsequent estimated total daily medicament value utilizing the updated trust value.

In a further aspect, a further medicament delivery system is provided. The medicament delivery system may include a wearable medicament delivery device, a memory storing programming instructions, and a processor. The processor may be operable to execute the programming instructions, which cause the processor to set a basal delivery rate at a preset rate without reference to a user's analyte history. After setting the basal delivery rate, the processor receives a user analyte history, and determines an extent of time spanned by the received analyte history. The processor, based on the extent of time spanned by the received analyte history, defines a duration for the basal delivery rate to be set at the preset rate.

A further medicament delivery system is provided in yet another aspect. The medicament delivery system may include a wearable medicament delivery device, a memory storing programming instructions, and a processor. The processor may be operable to execute the programming instructions, which cause the processor to receive an available medicament delivery history of a user, and determine whether the available medicament delivery history spans an extent of time greater than a first glucose history duration. The processor, in response to a determination that the extent of time spanned by the available medicament delivery history is greater than the first glucose history duration, determines an average daily medicament delivered. The processor calculates a trust value of the available medicament delivery history, and utilizes the trust value to determine a total daily medicament value of the user.

### BRIEF DESCRIPTION OF THE DRAWINGS

To easily identify the discussion of any particular element or act, the most significant digit or digits in a reference number refer to the figure number in which that element is first introduced.

FIG. 1 illustrates an exemplary medicament system operable to implement the examples disclosed herein.

FIG. 2 illustrates an exemplary process for a delivering medicament in the absence of analyte history.

FIG. 3 illustrates an exemplary process that determines and utilizes a trust value for an analyte history.

FIG. 4 illustrates another exemplary process that determines and utilizes a trust value for an analyte history.

FIG. 5 provides an exemplary graphic that illustrates modification of a total daily medicament value.

FIG. 6 illustrates an exemplary process for setting a basal delivery rate according to subject matter described herein.

## DETAILED DESCRIPTION

The following discussion describes a system, devices, and techniques that provides a beneficial resolution to questions, such as how is a medicament delivery system delivery schedule set when a user first starts using a wearable medicament delivery device, and how do we know what amount of medicament, such as insulin, to deliver to a user? For example, there may not be any history or feedback for the system to take advantage of when the user initially starts using the wearable medicament delivery device. It may not incur significant initial risk to users to set a daily dosage of insulin to be 20 Units (U) when no usage history is available, but such a high dosage may only be safe for a short period of time. However, the short period of time may not be long enough for the medicament delivery system to determine a user's response to the 20 U daily insulin dosage without any trustworthy amount of usage history.

The present disclosure provides a number of solutions for new users, and old users who have limited medicament delivery history that does not extend beyond a couple of days, or even part of a day.

The following discussion describes FIG. 1, which illustrates an exemplary medicament system operable to implement the examples disclosed herein.

The medicament system 100 is suitable for delivering a liquid medicament, such as insulin or the like, to a user in accordance with the disclosed embodiments. The medicament system 100 may include a wearable medicament device 102, a controller 104, and/or an analyte sensor(s) 106.

The medicament delivery system 100 may also include or engage with cloud-based services 110, a network 112, a computing device 114, a fitness device 116, a smart accessory device 118, and other wearable device(s) 120.

The wearable medicament delivery device 102 may be a wearable device that is worn on the body of the user. The wearable medicament delivery device 102 may be directly coupled to a user (e.g., directly attached to a body part and/or skin of the user via an adhesive, or the like). In an example, a surface of the wearable medicament delivery device 102 may include an adhesive to facilitate attachment to the skin of a user.

The wearable medicament delivery device 102 may include a processor 150, a housing(s) 108, a memory 142, a user interface 152, a pump 154, a reservoir 156, communication circuitry 158, an optional continuous glucose monitor 160b, and an optional ketone sensor 162b.

The processor 150 may be implemented in hardware, software, or any combination thereof, and may, for example, be a microprocessor, a logic circuit, a field programmable gate array (FPGA), an application specific integrated circuit (ASIC) or a microprocessor coupled to a memory. The processor 150 may maintain a date and time as well as be operable to perform other functions (e.g., calculations or the like). The processor 150 may be operable to execute a control application 146 stored in the memory 142 that enables the processor 150 to direct operation of the wearable medicament delivery device 102. The control application 146 may control insulin delivery to the user per an AMD control approach as describe herein. For example, the control application 146 may be an AMD algorithm that is operable to implement the functions described with reference to the examples of FIGs. 2-6. The memory 142 may hold settings 144 for a user, such as AMD algorithm settings, such as maximum insulin delivery, insulin sensitivity settings, target set points for glucose or ketones, control factor settings, total daily insulin (TDI) settings, and the like. The memory may also store other data 148, such as, total daily insulin values, ketone values and/or glucose measurement values from analyte sensor(s) 106 or controller 104, insulin dosage and/or delivery amounts (both basal and bolus), and the like from prior usage minutes, hours, days, weeks, or months.

The analyte sensor(s) 106 may be operable to collect physiological condition data, such as ketone values, ketone values with a time stamp, glucose measurement values (also referred to herein as "blood glucose values" or "blood glucose"), glucose measurement values and a timestamp, and the like. In some examples, the ketone values and/or the glucose values are shared with the wearable medicament delivery device 102, the controller 104, or both. In an additional example, the analyte sensor(s) 106 may include multiple sensors, such as a continuous glucose monitor 160a and a ketone sensor(s) 162a. In a further example, the wearable medicament delivery device 102 may optionally include a continuous glucose monitor 160b and a ketone sensor 162b, which may be removably incorporated into the one or more housing(s) 108, or be fully integrated within the wearable medicament device 102. For example, the continuous glucose monitor 160b and the ketone sensor 162b may be incorporated in one or more housing(s) 108 of the wearable medicament

delivery device 102. Note that ketones may also be detected using a breath sensor (which is not shown but which may be incorporated in the controller 104) or urine content sensor, and the outputs of which may be stored in the respective memories 118 and 112 via a user input of the ketone levels; however, a subcutaneous ketone sensor, such as 162a or 162b, gives more accurate information and is more continuous.

In an example, the communication circuitry 158 of the wearable medicament device 102 may be operable to communicate with the analyte sensor(s) 106 and the controller 104 as well as the fitness devices 116, smart accessory device 118, and other wearable device(s) 120. The communication circuitry 158 may be operable to communicate via Bluetooth, cellular communication, near field communication (NFC) and/or other wireless protocols. While not shown, the memory 142 may include a primary memory and a secondary memory that are operable to store different values over longer periods of time or shorter periods of time. The memory 142 may include random access memory (RAM), read only memory (ROM), optical storage, magnetic storage, removable storage media, solid state storage or the like.

The reservoir 156 of the wearable medicament device 102 may be operable to store liquid drugs, medicaments, or therapeutic agents suitable for automated delivery, such as, insulin, glucagon-like peptide-1 (GLP-1) receptor agonist, pramlintide, glucose-dependent insulinotropic polypeptide (GIP), glucagon, other hormones, or co-formulations of two or more of the foregoing; as well as pain relief drugs, such as opioids or narcotics (e.g., morphine, or the like), methadone, weight loss medicaments, blood pressure medicines, chemotherapy drugs, fertility drugs, or the like.

A fluid path to the user from the reservoir 156 and/or the pump 154 may be provided via tubing and a needle/cannula (not shown). The fluid path may, for example, include tubing coupling the wearable medicament device 102 to the user (e.g., via tubing coupling a needle or cannula to the reservoir 156). The wearable medicament device 102 may be operable based on control signals from the processor 150 to expel the liquid drugs, medicaments, or therapeutic agents, such as insulin, from the reservoir 156 to deliver doses of the drugs, medicaments, or therapeutic agents, such as the insulin, to the user via the fluid path. The processor 150 may be operable to cause insulin to be expelled from the reservoir 156.

There may be one or more communication links 164 with one or more devices physically separated from the wearable medicament device 102 including, for example, a controller 104 of the user and/or a caregiver of the user and/or analyte sensor(s) 106. Other communication links 140, 166 and 168 may be operable to communicatively couple devices within the medicament delivery system 100. The analyte sensor(s) 106 may, for example, communicate with the wearable medicament device 102 via a wireless communication link 140 and/or may communicate with the controller 104 via a wireless communication link 166. The communication links 140, 164, 166 or 168 may include any wired or wireless communication link operating according to any known communications protocol or standard, such as Bluetooth®, NFC, Wi-Fi, a near-field communication standard, a cellular standard, or any other wireless protocol.

The user interface 152 of the wearable medicament device 102 may also include an integrated display device for displaying information to the user, and in some embodiments, receiving information from the user. For example, the user interface 152 may include a touchscreen and/or one or more input devices, such as buttons, knob(s), microphone, or a touch-sensitive pad that enable a user to provide an input.

In addition, the processor 150 may be operable to receive data or information from the analyte sensor(s) 106 as well as other devices, such as 116, 118 and/or 120, that may be operable to communicate with the wearable medicament device 102.

The medicament system 100 may include an analyte sensor(s) 106 for sensing the levels of one or more analytes of a user. The analyte levels may be used as physiological condition data and be sent to the controller 104 and/or the wearable medicament delivery device 102. The sensor(s) 106 may be coupled to the user by, for example, adhesive or the like and may provide information or data on one or more medical conditions and/or physical attributes of the user. The sensor(s) 106 may be a continuous glucose monitor (CGM), ketone sensor, or another type of device or sensor(s) that provides glucose measurements and/or ketone measurements. The sensor(s) 106 may be physically separate from the wearable medicament delivery device 102 or may be an integrated component thereof. The sensor(s) 106 may provide the processor 130 and/or processor 150 with physiological condition data indicative of measured or detected glucose levels of the user. The information or data provided by the sensor(s) 106 may be used to modify a medicament delivery schedule and thereby cause the adjustment of medicament operations of the wearable medicament device 102.

The controller 104 of the medicament system 100 may include communication circuitry 122, a user interface 128, a processor 130, and a memory 132. In the depicted example, the controller 104 may include a processor 130 and a memory 132. The controller 104 may be a special purpose device, such as a dedicated personal diabetes manager (PDM) device. The controller 104 may be a specifically-programmed, general-purpose device that is a portable electronic device, such as any portable electronic device, smartphone, smartwatch, fitness device, tablet or the like including, for example, a dedicated processor, such as processor, a micro-processor or the like.

The memory 132 may store one or more applications, such as other data 134, a control application 136, and settings 138 of the AMD algorithm or the like as described herein. In addition, the memory 132 may be operable to store such as medicament history, glucose measurement values over a period of time, total daily insulin values, ketone values, and the like as other data 134. For example, the memory 132 is coupled to the processor 130 and operable to store

programming instructions, such as the control application 136 and settings 138, and data, such as other data 134, related to a glucose of a user and/or data related to an amount of insulin expelled by the wearable medicament delivery device 102.

The processor 130 may also be operable to execute programming code stored in the memory 132. For example, the memory 132 may be operable to store a control application 136, such as an AMD algorithm for execution by the processor 130 and/or similar programming code. The control application 136 may be operable to cause the processor 130 to control the wearable medicament delivery device 102, including the automated delivery of medicament, a drug, or therapeutic agent based on recommendations and instructions from the AMD algorithm, such as those recommendations and instructions described herein.

The controller 104 may include a user interface (UI) 128 for communicating with the user. The user interface 128 may include a display, such as a touchscreen, for displaying information. The touchscreen may also be used to receive input when it is a touch screen. The user interface 128 may also include input elements, such as a keyboard, button, knob or the like. The touchscreen display, under control of the processor 119, may be operable to, in response to the received input, generate a response to a user's treatment regimen as well as cause the presentation of prompts on a graphical user interface as described with reference to the later examples described with reference to FIGs. 2-6.

The controller 104 may interface via a wireless communication link of the wireless communication links 164 or the like with a network, such as a LAN or WAN or combination of such networks that provides one or more servers or cloud-based services 110 via communication circuitry 122. The communication circuitry 122, which may include transceivers 124 and 126, may be coupled to the processor 130. The communication circuitry 122 may be operable to transmit communication signals (e.g., command and control signals) to and receive communication signals (e.g., via transceivers 124 or 126) from the wearable medicament device 102 and the analyte sensor(s) 106 as well as devices 116, 118, and 120, computing device 114 and/or cloud-based services 110. In an example, the communication circuitry 122 may include a first transceiver, such as 124, that may be a Bluetooth transceiver, which is operable to communicate with the communication circuitry 122 of the wearable medicament device 102, and a second transceiver, such as 126, that may be a cellular or Wi-Fi transceiver operable to communicate via the network 112 with computing device 114 or with cloud-based services 110.

The wearable medicament device 102 and/or the controller 104 may interface with a network 112. The network 112 may include a local area network (LAN), a wide area network (WAN) or a combination therein. The computing device 114 may be interfaced with the network, and the computing device may communicate with the insulin delivery device 102. The computing device 114 may be a healthcare provider device or guardian device through which a user's controller 104 may interact to obtain or receive information, store or receive settings, and the like. The AMD algorithm operating, as or in cooperation with, the control application 120 may present a graphical user interface on the computing device 114 enabling the input and presentation of information related to the AMD algorithm and the example techniques and processes described herein.

The cloud-based services 110 may be operable to receive and store user history information, such as glucose measurement values and/or ketone measurement values over a set period of time (e.g., days, months, years), a medicament delivery history that includes insulin delivery amounts (both basal and bolus dosages) and insulin delivery times, types of insulin delivered, indicated meal times, glucose history, ketone history, glucose measurement value trends or excursions or other user-related diabetes treatment information, or the like.

Other devices, like smart accessory device 118 (e.g., a smartwatch or the like), fitness device 116, and other wearable device(s) 120 may be part of the medicament system 100. These devices may communicate with the wearable medicament device 102 to receive information and/or issue commands to the wearable medicament device 102. These devices 116, 118 and 120 may execute computer programming instructions to perform some of the control functions otherwise performed by processor 150 or processor 119. These devices 116, 118 and 120 may include user interfaces, such as touchscreen displays for displaying information such as current glucose level, medicament on board such as insulin on board, medicament history, or other parameters or treatment-related information and/or receiving inputs, such as those described with reference to the examples of FIGs. 2-6. These devices 116, 118 and 120 may also have wireless communication connections with the analyte sensor(s) 106.

Additionally, the processor 130 is operable to collect physiological condition data related to the user from sensors, such as the analyte sensor(s) 106 or heart rate data, for example, from the fitness device 116, other wearable device(s) 120, or the smart accessory device 118. In an example, the processor 130 executing the AMD algorithm may determine a dosage of medicament to be delivered based on the collected physiological condition of the user and/or a specific factor determined based on the subjective medicament need parameter. The processor 130 may output a control signal via one of the transceivers 126 or 124 to the wearable medicament delivery device 102. The outputted control signal may be received by communication circuitry 158 of the wearable medicament delivery device 102 and cause the processor 150 to deliver command signals to the pump 154 to deliver an amount of the determined dosage of medicament from the reservoir 156 to the user. The processor 130 may also be operable to perform calculations to update or establish settings of the AMD algorithm, such as glucose history, ketone history, medicament delivery history, total daily medicament (TDM), basal delivery dosages, and the like as discussed as herein. Modifications to the AMD algorithm settings, such as basal delivery dosages, TDM, or the like, may be stored in the memory 132, for example, as settings 138.

While functions were described with reference to the processor 130 of the controller 104, it is understood that the processor 150 of the wearable medicament delivery device 102 may also be operable to perform the similar functions as described herein.

While the medicament delivery system 100 is described with reference to delivery of medicament and the use of an AMD algorithm, the medicament delivery system 100 may be operable to implement a diabetes treatment regimen insulin and/or equivalents as described below.

In an example, a history of analyte values may be absent because the user is not using an analyte sensor, such as a ketone sensor or a continuous glucose monitor (CGM), because the user may be new to using wearable medical devices. Alternatively, the user may have had an analyte sensor(s), and the analyte values are not being received by the processor, the analyte sensor(s) is malfunctioning, or the like. Regardless of the situation, the present techniques and processes account for a lack of analyte history, such as glucose history and/or ketone history. One or more of the presently described techniques and processes also account for when a user first starts using an analyte sensor(s)and feedback not yet present at a starting period or very little history is available due to a brief period of time using the analyte sensor(s).

In an operational example, a processor of the AMD system when executing programming instructions may be operable to execute process 200 during an initializing phase of a wearable medicament delivery device. An example of an initialization phase may be when a user first begins receiving medicament from the wearable medicament delivery device until approximately 2 hours, 4 hours, 8 hours, 12 hours, 24 hours, 36 hours, 48 hours, 72 hours, or the like. In some embodiments, the initialization phase has a length between 2 hours to 7 days, more specifically 4 hours to 72 hours. In step 202, a processor, during the initializing phase of the wearable medicament delivery device, may be operable to deliver a first amount of medicament in absence of analyte values of the user for a first duration threshold, or in other words, when analyte values (such as glucose measurement values) are not being received by the wearable medicament delivery device 102 or associated device (such as controller 104). In an example, the first amount of medicament may be based on an assumption that the user's absent analyte levels are greater than a first threshold. For example, the first threshold may a hypoglycemia threshold, such as a glucose level of 70 mg/dL, or the like. As for the first duration threshold, it may be approximately 3 hours and may be measured from a start of the initialization phase. In some embodiments, the first duration threshold is between about 1 hour to 10 hours, more specifically between about 2 hours to about 6 hours and in particular between about 2.5 hours to 4 hours

The first amount of medicament, for example, may be a predetermined percentage (e.g., 10%-50%, or the like) over a clinically-determined amount of basal-delivered medicament for a person with diabetes. The clinically-determined amount of basal-delivery medicament is the basal-delivery amount determined by the user or a healthcare professional that may be based on particular physiological factors, such as the patient's weight, BMI, or others, or adapted over time based on the user's manual titration that best matched their preferences for diabetes care. Alternatively, the first amount of medicament may be a reduced basal medicament delivery rate of an AMD algorithm that is reduced by a predetermined percentage (e.g., 5%-50%, or the like). For example, an AMD algorithm that has had no inputs, for example, while being initialized, may deliver a first amount of medicament, such as no more than 5 U TDI, or an amount of basal medicament at a minimum acceptable medicament delivery rate, such as 0.1 U/h, as an initial delivery rate when no historical data are available.

Specifically, in the case of a person with diabetes, an AMD system may deliver up to 50% increased basal for up to 6 hours without incurring significant harm, and up to 100% increased basal without incurring more than an overdose low situation. An overdose low situation is an instance of hypoglycemia that does not require medical intervention. By delivering no more than the 100% increased basal, instances of hypoglycemia requiring medical intervention can be avoided. In particular, by infusing only up to 100% increased basal, blood glucose measurements from which more appropriate basal rates can be calculated may become available prior to the patient arriving at an hypoglycemic situation requiring medical intervention. Therefore, the AMD system may provide insulin delivery rate up to 0.2 U/h for 3 hours to 6 hours, depending on duration of prior available history, as follows:

Table 1.

| Duration of no- input insulin delivery history | 0-3 hours | 3-6 hours | 6+ hours |
|---|---|---|---|
| Option 1 | 0.15 U/h | 0.15 U/h | 0.1 U/h |
| Option 2 | 0.2 U/h for variable duration *D* depending on extent of available history, then 0.15 U/h | | 0.1 U/h |

$$D = 3 + 3\min\left(\frac{H}{12}, 1\right)$$

Where H = the number of hours of available previous history

In addition, the clinically defined thresholds of basal delivery for risk of hyperglycemia and hypoglycemia can be utilized to assess maximum possible delivery rates when given no historical data and assuming a minimum insulin need of 0.1 U/h. For example, hyperglycemia and hypoglycemia thresholds can be defined based on a potential hazard (e.g., hypoglycemia and/or hyperglycemia) associated with a specific situation. The severity of hyperglycemia and hypoglycemia may be a result of different degrees of sub-optimal insulin over- or under-delivery. This severity may also be associated with different levels of risk to the user, such as low risk for a low degree of hyperglycemia and hypoglycemia, and vice versa. In an example, the processor may be operable to evaluate various hyperglycemia and hypoglycemia thresholds and the situation. For example, if there is no harm and the user's glucose level is greater than a first threshold, such as a hypoglycemic threshold, the AMD algorithm may be expected to deliver an amount of medicament that substantially keeps the user's glucose levels within a present range of the user's target glucose setpoint.

In step 204, process 200 at an end of the first duration threshold, the processor determines the analyte values continue to be absent. For example, the processor may query a memory for updated analyte values, or may poll communication circuitry for a connection to an analyte sensor.

In step 206, process 200 delivers a second amount of medicament for a second duration threshold. The second duration threshold may be greater than the first duration threshold. In some examples, the second amount of medicament is different from the first amount of medicament. The second amount of medicament may be based on an assumption that the user's absent analyte levels are still greater than the first threshold. In an example, the second amount of medicament is a predetermined percentage over a clinically-determined amount of basal-delivered medicament for a person with diabetes and based on the second duration threshold. With regard to the second duration threshold, the second duration threshold, similar to the first duration threshold, may be measured from a start of the initialization phase and may be approximately 6 hours, approximately 8 hours, a multiple of the first duration threshold, or the like. In some embodiments, the second duration threshold is between about 2 hour to 20 hours, more specifically between about 4 hours to about 12 hours and in particular between about 5 hours to 8 hours.

In a further step, the processor may, at an end of the second duration threshold, determine whether analyte values continue to be absent. In response to the continued absence of the analyte values, the processor may determine a third amount of medicament for delivery by the wearable medicament delivery device.

In yet a further step, the processor may be operable to receive a set of analyte values of the user. Based on the set of analyte values, the processor may estimate a total daily medicament value needed to maintain the user's analyte levels above the first threshold. Based on the estimated total daily medicament value to provide a third amount of medicament, the processor may adjust the second amount of medicament for delivery by the wearable medicament delivery device.

Additionally, the processor when estimating the total daily medicament value needed to maintain the user's analyte levels above the first threshold, may be operable to determine a medicament correction value based on the received set of analyte values. The processor may use the medicament correction value to adjust the total daily medicament value. The determined medicament correction value may be the additional medicament amount that would have been delivered to maintain the analyte value at the target.

As an alternate example, if there is a starting total daily medicament (TDM) value that is based on the user's inputs that are roughly correlated with the user's insulin needs - such as the user's weight - a fraction of this starting TDM value may be given until sufficient history is provided, such as 25% of this assumed TDM. For example, if the TDM calculated based on the user's weight is 30 U, and the system does not have glucose feedback and/or has insufficient or no historical data (e.g., glucose, ketone, or medicament delivery), the AMD algorithm may cause medicament delivery based on 25%, or 7.5 U, as basal medicament delivery.

FIG. 3 illustrates an exemplary process that determines and utilizes a trust value for an analyte history. When determining a trust value, the processor, when implementing process 300, may receive a glucose history of a user having a first glucose history duration (302).

In step 304, the processor may set a trust value based on the first glucose history duration. The trust value may be a percentage that ranges from 0 to 100. The trust value corresponds to an extent of a glucose history duration. For example, the trust value may be 0% if the glucose history duration is less than 4 hours and 100% of the glucose history duration is more than 72 hours. Values between 4 hours and 72 hours may be scaled, for example, per a linear scale. For example, given the previous example and a linear scaling, a first glucose history duration of 34 hours may result in a trust value of 50%.

In step 306, process 300 calculates an estimated total daily medicament value utilizing the trust value. The processor may be operable to calculate a basal dosage based on the estimated total daily medicament value.

In step 308, process 300 receive an updated glucose history of the user having a second glucose history duration.

In step 310, process 300 sets an updated trust value based on the second glucose history duration.

In step 312, process 300 calculates a subsequent estimated total daily medicament value utilizing the updated trust value. calculating an updated basal dosage based on the subsequent estimated total daily medicament value.

FIG. 4 illustrates another exemplary process that determines and utilizes a trust value for an analyte history. In step 402, the processor when executing process 400 receives an available medicament delivery history of a user. The

available medicament delivery history may include a history of the amount of medicament that has been delivered over a period of time, such as the last 1-48 hours or the like. If the wearable medicament delivery device is being used for a first time by a user (i.e., an initial use), the medicament delivery history may have no data. Alternatively, if the user has been using a wearable medicament delivery device for some time, the available medicament delivery history may include tens or hundreds of hours of data.

The data included in the medicament delivery history may include a number of units of medicament delivered during each control cycle of a wearable medicament delivery device, a number of units of medicament delivered during a 24 hour period, 12 hour period or the like, timestamp data for each medicament delivery, a number of units of medicament delivered as bolus dosages, and the like. In some examples, the medicament delivery history includes more data or less data than the foregoing list.

In step 404, the processor determines whether the available medicament delivery history spans an extent of time greater than a first glucose history duration.

As the processor executes process 400, the processor at step 406, in response to a determination that the extent of time spanned by the available medicament delivery history is greater than the first glucose history duration, determines an average daily medicament delivered.

In step 408, process 400 calculates a trust value of the available medicament delivery history. The processor may be operable to generate a higher trust value as the extent of time spanned by the available medicament delivery history increases beyond the first glucose history duration.

The processor, at step 410, may utilize the trust value to determine a total daily medicament value of the user.

For example, the processor may be operable to determine, based on the total daily medicament value, an amount of medicament to be delivered to the user by basal delivery via a wearable medicament delivery device.

FIG. 5 provides an exemplary graphic that illustrates modification of a total daily medicament value based on the process 400 of FIG. 4.

It is envisioned that the AMD system is operable to scale a user's medicament delivery rate, taking into account minimal previous history of prior analyte control outcomes and prior medicament delivery. The graphic 500 illustrates an example of how an AMD algorithm modifies a user's total daily medicament value based on the evaluation of a user's history of prior analyte control outcomes and prior medicament delivery.

A person with diabetes, for example, may begin use of a wearable medicament delivery device without having a history of prior analyte control outcomes and prior medicament delivery. As such, the AMD algorithm needs to begin delivering some level of medicament.

As discussed herein, a history of prior analyte control outcomes may be a glucose history and/or a ketone history that is based on the output of an analyte sensor. In an example, a glucose history refers to glucose measurement values taken by a continuous glucose monitor (CGM) and/or a blood glucose meter (e.g., a glucose level is obtained by finger stick methodology). For example, a glucose measurement value (and, perhaps other data, such as timestamp, trend information, prior glucose measurement value(s), or the like), may be obtained from a CGM approximately every 5 minutes. In a further example, the analyte sensor may be a ketone sensor, which may also be operable to provide a ketone value to a processor approximately every 5 minutes, or the like.

The AMD algorithm may use either the glucose values stored as a glucose history or the ketone values stored as a ketone history, or both, in the evaluation of the user's glycemic health and subsequent determine of total daily medicament value. The glucose history may include the data from a number of glucose values, which are actually glucose control outcomes (i.e., a result of the AMD algorithm causing delivery of a dosage of medicament). For example, an AMD algorithm may determine, based on the user's glucose history, a total daily medicament (TDM) value of *X* Units (U) of a medicament to be delivered to the user over the course of a day. The determination of the TDM value in response to the user's glucose level may be considered a glucose control decision. A change (or even no change) of the user's glucose level in response to the glucose control decision is considered a glucose control outcome.

However, in the absence of a glucose history and/or medicament delivery history that is greater than the first duration threshold, the processor may initial delivery of medicament at a predetermined dosage or amount. In the example of FIG. 5, the total daily medicament value is total daily insulin (TDI), where TDI_f is a final TDI determined by the AMD algorithm. When the glucose history and/or medicament delivery history as indicated by the number of number of operational cycles (i) is less than 144 operational cycles, or 12 hours, the initial value 502 may be set at a known clinically-safe value for a total daily medicament value. The clinically-safe value may be a value determined as safe by a healthcare professional, or a value that a user may manually have set to match the user's manual titration that best matches their preferences in diabetes care. A known clinically-safe value of a total daily medicament value, which in the example of FIG. 5 is TDI, may be 5 U. The AMD algorithm may set a TDI_f value to initial value 502 of 5 U, when the respective history is less than the 12 hours or 144 operational cycles.

Once the respective history, be it the glucose history, the ketone history, the medicament delivery history or a combination thereof, is collected, the scaling value 504 is applied according to a trust value. The trust value is based on the amount of respective history is collected. The more respective history that is collected the higher the trust value.

For example, the AMD system may be operable to collect glucose history, ketone history, and/or medicament delivery history from an analyte sensor or multiple analyte sensors, such as a glucose monitor, continuous glucose monitor, a ketone sensor, for a first duration 510. The first duration 510 may be measured in time (i.e., a first duration threshold) or in operational cycles (e.g., first duration of operational cycles).

An operational cycle of a AMD system, as used herein, is when a wearable medicament delivery device delivers medicament as basal dosage that is determined by the AMD algorithm to a user. The operational cycle includes, in addition to the delivery of medicament, obtaining glucose measurement values from a CGM, obtaining ketone measurement value from a ketone sensor, storing the glucose measurement values and/or ketone measurement values. An operational cycle may be approximately 5 minutes in duration. During the 5-minute operational cycle, the processor may receive a glucose measurement value, a ketone measurement value, or more frequently, by a wearable medicament delivery device from a CGM. The 5-minute interval of an operational cycle of the wearable medicament delivery device may, in addition to obtaining a glucose value and delivering medicament encompass the foregoing actions as well as others, such as, calculating a new medicament dosage for a next operational cycle, storing medicament delivery information and glucose measurement values, ketone values, and the like).

To provide some context for a duration, such as first duration 510, that may be considered by a processor executing the processes described herein, the number of 5-minute operational cycles in a 1-hour (i.e., 60 minutes) period is 12. As such, the first duration 510 may be determined either in operational cycles (e.g., 12) or in time (e.g., hours). For example, the number of operational cycles, in a 12-hour period of time is 144; in a 24-hour period of time is 288; in a 36-hour period of time is 432; and, in a 48-hour period of time is 576. Further, the time durations may also not be limited to discrete 12-hour increments, but can be other time durations, such as 14-hours (168 operational cycles), 17-hours (204 operational cycles), 29-hours (348 operational cycles), 33-hours (396 operational cycles), 46-hours (552 operational cycles), or the like. It is also envisioned that the first duration threshold may include fractions of hours, such as 10-minutes, 15-minutes, 20-minutes, multiples thereof, or the like. Whatever the number of operational cycles, the duration of time over which data is collected for each operational cycle is compared to a duration threshold, such as the first duration threshold, which may be 12-hours, 16-hours, 24-hours, or the like. In some embodiments, the duration threshold may be between about 6 hours to about 48 hours, more specifically between about 12 hours to about 24 hours.

When the duration of the medicament delivery history, glucose history, and/or ketone history exceeds the first duration 510, the processor begins scaling TDI_f value as shown by scaling value 504. The scaling value 504 may increase as the respective history (i.e., glucose history, ketone history, medicament delivery history, or combinations thereof) becomes more extensive; however, the scaling value 504 may be limited according to a trust value determined for the duration of the medicament delivery history, glucose history, and/or ketone history. The scaling value 504 may be applied for a set period of time that extends for a second duration threshold. When the respective duration of the medicament delivery history, glucose history, ketone history, or combination thereof exceeds a second duration, the scaling value 504 may no longer be applied, and the processor may fully trust the respective history.

As discussed above with reference to earlier examples, the processor executing an AMD algorithm may evaluate whether a glucose history, ketone history, and/or medicament delivery history is available for a duration of time that exceeds a first duration threshold, such as 12 hours, or the like. For example, after the TDI_f is set at the initial value 502, the AMD algorithm may cause the executing processor to begin monitoring a memory (and/or cloud-based service) in which user's ketone history or glucose history may be stored.

Once a respective history exceeds the first duration and the scaling value 504 is no longer applied, the AMD algorithm may utilize a value, TDI_est(*i*) to determine an amount of insulin to be delivered to the user as a fully scaled value 506. For example, when the number of operational cycles exceeds 576 or 48 hours, the AMD algorithm may utilize just the TDI_est(*i*) value as the fully scaled value 506.

Alternatively, if a minimal duration of glucose history (i.e., a number of hours of glucose measurement values), such as between 12 to 48 hours, is available, the AMD system may begin utilizing the glucose control outcomes and previous insulin delivery history in a scaling manner by proceeding directly to determining a user's TDI_f(*i*) an initial value (with history) 508. For example, the processor may determine an TDI_est(*i*) that is used as TDI_f(*i*).

The above general description of FIG. 5 may be implemented in a number of different ways. The following discussion provides an example implementation for determining the scaling value 504 as well as the fully scaled value 506 and/or the initial value (with history) 508.

In an example implementation, a component of the scaling of the total daily medicament value may be an estimated total daily medicament value, which in the example of FIG. 5 is an estimated TDI, $TDI_{est}(i)$, as shown in Equation 2 below. The $TDI_{est}(i)$ may be calculated based on prior insulin delivery history and glucose outcomes as follows:

$$TDI_{est}(i) = 288\frac{\sum_{k=1}^{i} I(i)}{i} + \frac{\overline{CGM}(i) - target(i)}{\frac{1600}{288\frac{\sum_{k=1}^{i} I(i)}{i}}}$$

where 288 is the number of operational cycles in a 24 hour period of time, the variable *i* is a current operational cycle number of a wearable medicament delivery device and/or CGM, $\overline{CGM}(i)$ is an average CGM value at the current operational cycle *i, target (i)* is the target glucose setpoint of the ADM algorithm at the current operational cycle (*i*), and the denominator of the second term is a correction factor (i.e., 1600 divided by an average insulin that would be delivered over 12 hours). The correction factor could be any other known correction factor, such as 1800 Rule or 1500 Rule. The "288" could also be another number, for example 144, if the frequency of operational cycles is 10 minutes.

The operational cycle represented variable (*i*) in Equation 2 may begin incrementing as soon as a user starts using a wearable medicament delivery device. The value of the operational cycle may start (or restart) at zero, or may continue from a last value of (*i*) (for example, if a wearable medicament delivery device is replaced with another), if either a glucose history or a medicament delivery history, or both are available. For example, the user may have begun using a wearable medicament delivery device a year (365 days) ago, and, with 288 operational cycles in a day, the value of (*i*) could be over 100,000. However, an extensive medicament delivery history may not be available for the experienced user for various reasons, such as a switch from one manufacturer's wearable medicament delivery device to another, lost data, corrupted data, incompatible data (e.g., data format may not correct or usable), or the like.

The first term of Equation 2 determines an average amount of medicament (i.e., insulin ($I(i)$) that has been delivered over a 24-hour period. The number of insulin deliveries over a 24-hour period of time is 288 since a wearable medicament delivery device makes 12 deliveries per hour. Note that the summation does not have to be for a full 24-hour period of time.

The second term of Equation 2 calculates the corrected average deviation from the target glucose setpoint. The second term utilizes the average CGM value at each current operational cycle *i* from which is subtracted the user's target glucose setpoint (i.e., *target*(*i*)). This average deviation value is then "corrected" utilizing a standard correction factor calculation, which, in this case, uses the 24-hour average insulin delivered that was calculated in the first term. The result of Equation 2 is the estimated total daily medicament value $TDI_{est}(i)$, where the medicament is insulin. In Equation 2, the $TDI_{est}(i)$ has been determined based on a relatively small sample set of analyte values and a small sample set of the amount of medicament delivered over the period of time values may then be further used to determine a more likely, or more "trusted" TDI value, because smaller sample sizes (e.g., 144 operational cycles) of user history are less trustworthy than the larger sample sizes (e.g., 576 operational cycles).

Equation 2 above provides an estimate of TDI for a user at the given time or at a given operational cycle.

To account for the level of "trust" of this TDI estimate, Equation 3 may be used, which enables scaling the trust in the user history from 0 to 100% based on the duration of history or number of available cycles of history, from 12 to 48 hours (144 to 576 cycles) as shown in Equation 3:

$$TDI_f(i) = \left(1 - \max\left(0, \min\left(1, \frac{i-144}{432}\right)\right)\right) \cdot 5 + \max\left(0, \min\left(1, \frac{i-144}{432}\right)\right) TDI_{est}(i)$$

where $TDI_{est}(i)$ is the estimated total daily medicament value that is calculated as above in Equation 3.

As shown in Equation 3, once the medicament delivery system has more than 48 hours of history (i.e., 576 operational cycles), the AMD algorithm may fully trust the TDI calculation.

The system may utilize the $TDI_f(i)$ value as in Equation 4 to determine an estimated hourly basal dosage for the user as follows:

$$basal_f(i) = \frac{TDI_f(i)}{48},$$

where 48 represents the division of TDI equally between basal dosages and bolus dosages over a period of 24 hours.

FIG. 6 illustrates an exemplary process for setting a basal delivery rate according to subject matter described herein. The process 600 may be utilized when a user first begins use of a wearable medicament delivery device that is operable to cooperate with an analyte sensor. In step 602, the process 600 causes a processor to set a basal delivery rate at a preset rate without reference to a user's analyte history. For example, the basal rate may be set according to the

graphic 500 of FIG. 5. At step 604, the processor, after setting the basal delivery rate, may receive a user's analyte history. For example, the processor may access or query a memory or cloud-based service to determine whether a user history, such as a glucose history and/or a ketone history, is available or, if an analyte sensor has begun delivering glucose measurement values or ketone measurement values.

In step 606, the processor may determine a duration spanned by the received analyte history. The processor may evaluate a timestamp that is provided with either glucose measurement values or ketone measurement values to determine whether the duration of the analyte history met a first duration threshold. The first duration threshold may be a period of time spanned by the received analyte history that is measured in hours or is measured in a number of operational cycles. Either of these durations (i.e., time or number of operational cycles) is the as those durations discussed with reference to FIG. 5.

Based on the duration spanned by the received analyte history, the processor as it executes process 600 defines a set time period for the basal delivery rate to be set at the preset rate (608). For example, the processor, when defining the set time period for the basal delivery rate, is operable to define the set time period as a preset value plus a fraction of the preset value, and the fraction of the preset value is determined based on the duration spanned by the received analyte history. The processor may, for example, utilize option 2 of Table 1 above when setting the set time period.

Software related implementations of the techniques described herein, such as the processes examples described with reference to the above discussion and figures may include, but are not limited to, firmware, application specific software, or any other type of computer readable instructions that may be executed by one or more processors. The computer readable instructions may be provided via non-transitory computer-readable media. The various elements of the processes described with reference to the figures may be implemented in devices, apparatuses or systems that may include various hardware elements, software elements, or a combination of both. Examples of hardware elements may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, processes, procedures, software interfaces, application program interfaces (API), instruction sets, computing code, computer code, code segments, computer code segments, words, values, symbols, or any combination thereof.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein. The present disclosure furthermore relates to computer programs comprising instructions (also referred to as computer programming instructions) to perform the aforementioned functionalities. The instructions may be executed by a processor. The instructions may also be performed by a plurality of processors for example in a distributed computer system. The computer programs of the present disclosure may be for example preinstalled on, or downloaded to the medicament delivery device, management device, fluid delivery device, e.g. their storage.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the numerous examples described herein were not mutually exclusive and may exist in various combinations and permutations, even if such combinations or permutations were not made express herein, without departing from the spirit and scope of the disclosed examples. In fact, variations, modifications, and other implementations

of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but by the claims appended hereto. Future filed applications claiming priority to this application may claim the disclosed subject matter in a different manner and may include any set of one or more features as variously disclosed or otherwise demonstrated herein.

Although the present invention is defined in the attached claims, it should be understood that the present invention can also (alternatively) be defined in accordance with the following embodiments:

1. A medicament delivery system, comprising:

    a wearable medicament delivery device;
    a memory storing programming instructions;
    a processor operable to execute the programming instructions, which cause the processor to:

        during an initializing phase of the wearable medicament delivery device, deliver a first amount of medicament in absence of analyte values of a user for a first duration threshold, wherein the first amount of medicament is based on an assumption that the absent analyte levels are greater than a first threshold;
        at an end of the first duration threshold, determine the analyte values continue to be absent; and
        deliver a second amount of medicament for a second duration threshold based on the determination, wherein the second amount of medicament is different from the first amount of medicament and based on an assumption that the user's absent analyte levels are still greater than the first duration threshold.

2. The medicament delivery system of embodiment 1, wherein the first amount of medicament is a predetermined percentage over a clinically-determined amount of basal-delivered medicament for a person with diabetes.

3. The medicament delivery system of embodiment 1, wherein the second amount of medicament is a predetermined percentage over a clinically-determined amount of basal-delivered medicament for a person with diabetes and based on the second duration threshold.

4. The medicament delivery system of embodiment 1, wherein the processor is operable to:

    receive a set of analyte values of the user;
    based on the set of analyte values, estimate a total daily medicament value needed to maintain the user's analyte levels above the first threshold; and
    adjust the second amount of medicament based on the estimated total daily medicament value to provide a third amount of medicament for delivery by the wearable medicament delivery device.

5. The medicament delivery system of embodiment 4, wherein the processor, when estimating the total daily medicament value needed to maintain the user's analyte levels above the first threshold, is operable to:

determine a medicament correction value based on the received set of analyte values; and
adjust the total daily medicament value using the medicament correction value.

6. The medicament delivery system of embodiment 1, wherein the first duration threshold is approximately 3 hours, wherein the first duration threshold is measured from a start of the initialization phase.

7. The medicament delivery system of embodiment 1, wherein the second duration threshold is measured from a start of the initialization phase and is approximately 6 hours.

8. The medicament delivery system of embodiment 1, wherein the processor is further operable to:

at an end of the second duration threshold, determine whether the analyte values continue to be absent; and
in response to the continued absence of the analyte values, determine a third amount of medicament for delivery by the wearable medicament delivery device.

9. The medicament delivery system of embodiment 1, wherein the first threshold is a hypoglycemia threshold.

10. A medicament delivery system, comprising:

a wearable medicament delivery device;
a memory storing programming instructions;
a processor operable to execute the programming instructions, which cause the processor to:

receive a glucose history of a user having a first glucose history duration;
set a trust value based on the first glucose history duration;
calculate an estimated total daily medicament value utilizing the trust value;
receive an updated glucose history of the user having a second glucose history duration;
set an updated trust value based on the second glucose history duration; and
calculate a subsequent estimated total daily medicament value utilizing the updated trust value.

11. The medicament delivery system of embodiment 10, wherein the trust value is a percentage that ranges from 0 to 100.

12. The medicament delivery system of embodiment 10, wherein the trust value corresponds to an extent of a glucose history duration.

13. The medicament delivery system of embodiment 10, further comprising:
calculating a basal dosage based on the estimated total daily medicament value.

14. The medicament delivery system of embodiment 10, further comprising:
calculating an updated basal dosage based on the subsequent estimated total daily medicament value.

15. A medicament delivery system, comprising:

a wearable medicament delivery device;
a memory storing programming instructions;
a processor operable to execute the programming instructions, which cause the processor to:

set a basal delivery rate at a preset rate without reference to a user's analyte history;
after setting the basal delivery rate, receive an analyte history of the user;
determine a duration spanned by the received analyte history; and
based on the duration spanned by the received analyte history, define a set time period for the basal delivery rate to be set at the preset rate.

16. The medicament delivery system of embodiment 15, wherein the duration spanned by the received analyte history is measured in hours or a number of operational cycles.

17. The medicament delivery system of embodiment 15, wherein:

the processor, when defining the set time period for the basal delivery rate, is operable to define the set time period as a preset value plus a fraction of the preset value, and
the fraction of the preset value is determined based on the duration spanned by the received analyte history.

18. A medicament delivery system, comprising:

a wearable medicament delivery device;
a memory storing programming instructions;
a processor operable to execute the programming instructions, which cause the processor to:

receive an available medicament delivery history of a user;
determine whether the available medicament delivery history spans an extent of time greater than a first glucose history duration;
in response to a determination that the extent of time spanned by the available medicament delivery history is greater than the first glucose history duration, determine an average daily medicament delivered;
calculate a trust value of the available medicament delivery history; and
utilize the trust value and the average daily medicament delivered to determine a total daily medicament value of the user.

19. The medicament delivery system of embodiment 18, wherein the processor is further operable to:
determine, based on the total daily medicament value, an amount of medicament to be delivered to the user by basal delivery via the wearable medicament delivery device.

20. The medicament delivery system of embodiment 18, wherein the processor, when calculating the trust value of the available medicament delivery history, is further operable to:
generate a higher trust value as the extent of time spanned by the available medicament delivery history increases beyond the first glucose history duration.

## Claims

**1.** A medicament delivery system, comprising:

a wearable medicament delivery device;
a memory storing programming instructions;
a processor operable to execute the programming instructions, which cause the processor to:

during an initializing phase of the wearable medicament delivery device, deliver a first amount of medicament in absence of analyte values of a user for a first duration threshold, wherein the first amount of medicament is based on an assumption that the absent analyte levels are greater than a first threshold;
at an end of the first duration threshold, determine the analyte values continue to be absent; and
deliver a second amount of medicament for a second duration threshold based on the determination, wherein the second amount of medicament is different from the first amount of medicament and based on an assumption that the user's absent analyte levels are still greater than the first duration threshold.

**2.** The medicament delivery system of claim 1, wherein the first amount of medicament is a predetermined percentage over a clinically-determined amount of basal-delivered medicament for a person with diabetes; and/or,
wherein the second amount of medicament is a predetermined percentage over a clinically-determined amount of basal-delivered medicament for a person with diabetes and based on the second duration threshold.

**3.** The medicament delivery system of claim 1 or 2, wherein the processor is operable to:

receive a set of analyte values of the user;
based on the set of analyte values, estimate a total daily medicament value needed to maintain the user's analyte levels above the first threshold; and
adjust the second amount of medicament based on the estimated total daily medicament value to provide a third amount of medicament for delivery by the wearable medicament delivery device.

4. The medicament delivery system of claim 3, wherein the processor, when estimating the total daily medicament value needed to maintain the user's analyte levels above the first threshold, is operable to:

determine a medicament correction value based on the received set of analyte values; and
adjust the total daily medicament value using the medicament correction value.

5. The medicament delivery system of any one of claims 1 to 4, wherein the first duration threshold is between about 1 hour to 10 hours, more specifically between about 2 hours to about 6 hours and in particular between about 2.5 hours to 4 hours, wherein the first duration threshold is measured from a start of the initialization phase; and/or wherein the second duration threshold is measured from a start of the initialization phase and is between about 2 hour to 20 hours, more specifically between about 4 hours to about 12 hours and in particular between about 5 hours to 8 hours.

6. The medicament delivery system of any one of claims 1 to 5, wherein the processor is further operable to:

at an end of the second duration threshold, determine whether the analyte values continue to be absent; and
in response to the continued absence of the analyte values, determine a third amount of medicament for delivery by the wearable medicament delivery device.

7. The medicament delivery system of any one of claims 1 to 6, wherein the first threshold is a hypoglycemia threshold.

8. A medicament delivery system, comprising:

a wearable medicament delivery device;
a memory storing programming instructions;
a processor operable to execute the programming instructions, which cause the processor to:

receive a glucose history of a user having a first glucose history duration;
set a trust value based on the first glucose history duration;
calculate an estimated total daily medicament value utilizing the trust value;
receive an updated glucose history of the user having a second glucose history duration;
set an updated trust value based on the second glucose history duration; and
calculate a subsequent estimated total daily medicament value utilizing the updated trust value.

9. The medicament delivery system of claim 8, wherein the trust value corresponds to an extent of a glucose history duration.

10. The medicament delivery system of claim 8, further comprising:

calculating a basal dosage based on the estimated total daily medicament value; and/or
calculating an updated basal dosage based on the subsequent estimated total daily medicament value.

11. A medicament delivery system, comprising:

a wearable medicament delivery device;
a memory storing programming instructions;
a processor operable to execute the programming instructions, which cause the processor to:

set a basal delivery rate at a preset rate without reference to a user's analyte history;
after setting the basal delivery rate, receive an analyte history of the user;
determine a duration spanned by the received analyte history; and
based on the duration spanned by the received analyte history, define a set time period for the basal delivery rate to be set at the preset rate.

12. The medicament delivery system of claim 11, wherein:

the processor, when defining the set time period for the basal delivery rate, is operable to define the set time period as a preset value plus a fraction of the preset value, and
the fraction of the preset value is determined based on the duration spanned by the received analyte history.

**13.** A medicament delivery system, comprising:

a wearable medicament delivery device;
a memory storing programming instructions;
a processor operable to execute the programming instructions, which cause the processor to:

receive an available medicament delivery history of a user;
determine whether the available medicament delivery history spans an extent of time greater than a first glucose history duration;
in response to a determination that the extent of time spanned by the available medicament delivery history is greater than the first glucose history duration, determine an average daily medicament delivered;
calculate a trust value of the available medicament delivery history; and
utilize the trust value and the average daily medicament delivered to determine a total daily medicament value of the user.

**14.** The medicament delivery system of claim 13, wherein the processor is further operable to:
determine, based on the total daily medicament value, an amount of medicament to be delivered to the user by basal delivery via the wearable medicament delivery device.

**15.** The medicament delivery system of claim 13 or 14, wherein the processor, when calculating the trust value of the available medicament delivery history, is further operable to:
generate a higher trust value as the extent of time spanned by the available medicament delivery history increases beyond the first glucose history duration.

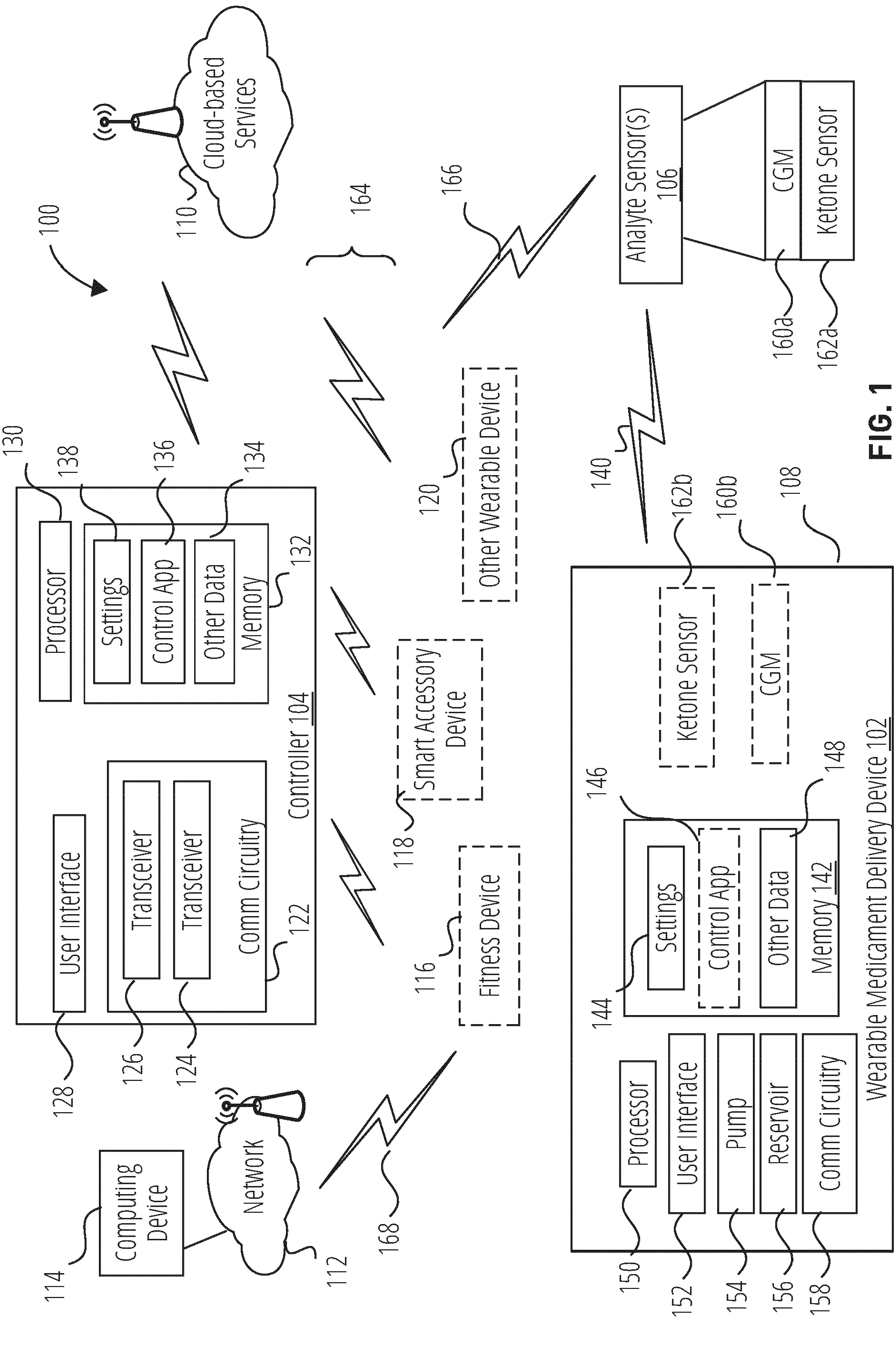
100
110
Cloud-based Services
164
166
Analyte Sensor(S) 106
CGM
Ketone Sensor
160a
162a
130
138
136
134
Processor
Settings
Control App
Other Data
Memory
132
Controller 104
User Interface
Transceiver
Transceiver
Comm Circuitry
122
128
126
124
120
Other Wearable Device
140
162b
160b
108
Smart Accessory Device
118
Fitness Device
116
Ketone Sensor
CGM
146
148
144
Settings
Control App
Other Data
Memory 142
Processor
User Interface
Pump
Reservoir
Comm Circuitry
Wearable Medicament Delivery Device 102
150
152
154
156
158
114
Computing Device
Network
112
168

FIG. 1

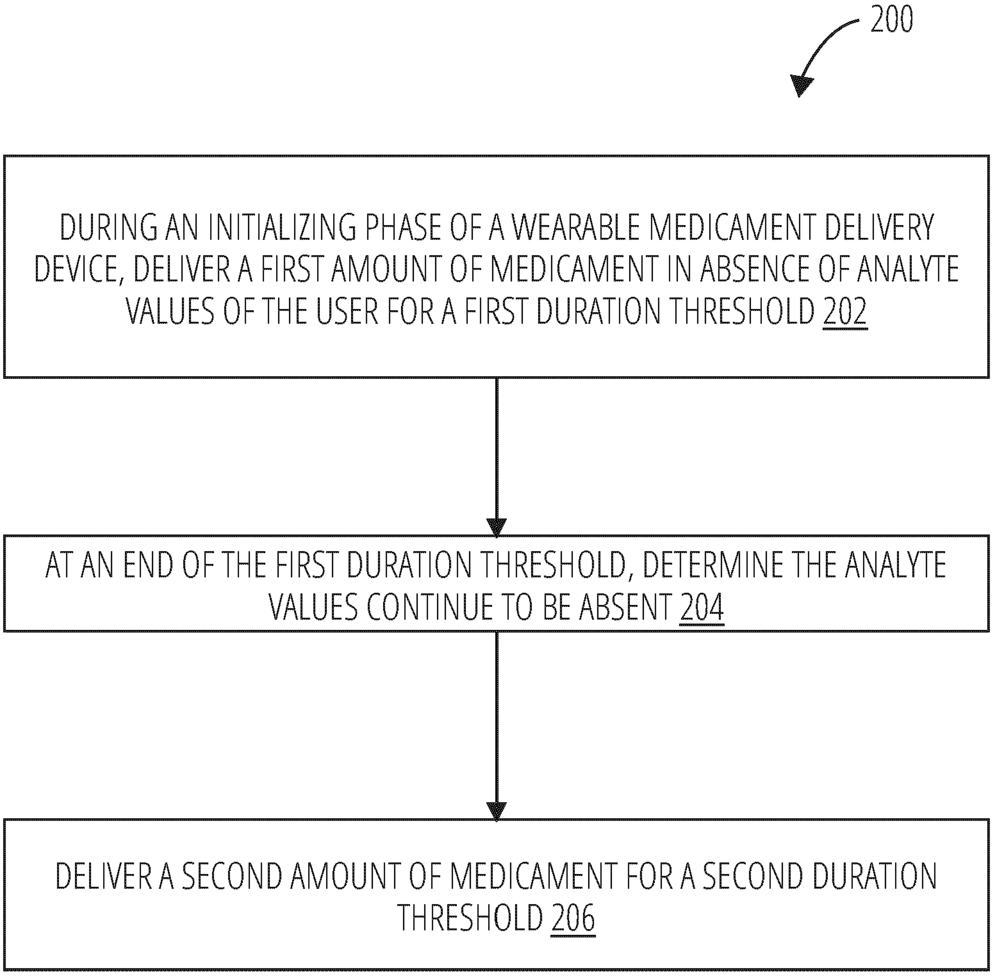
200
DURING AN INITIALIZING PHASE OF A WEARABLE MEDICAMENT DELIVERY DEVICE, DELIVER A FIRST AMOUNT OF MEDICAMENT IN ABSENCE OF ANALYTE VALUES OF THE USER FOR A FIRST DURATION THRESHOLD 202
AT AN END OF THE FIRST DURATION THRESHOLD, DETERMINE THE ANALYTE VALUES CONTINUE TO BE ABSENT 204
DELIVER A SECOND AMOUNT OF MEDICAMENT FOR A SECOND DURATION THRESHOLD 206

FIG. 2

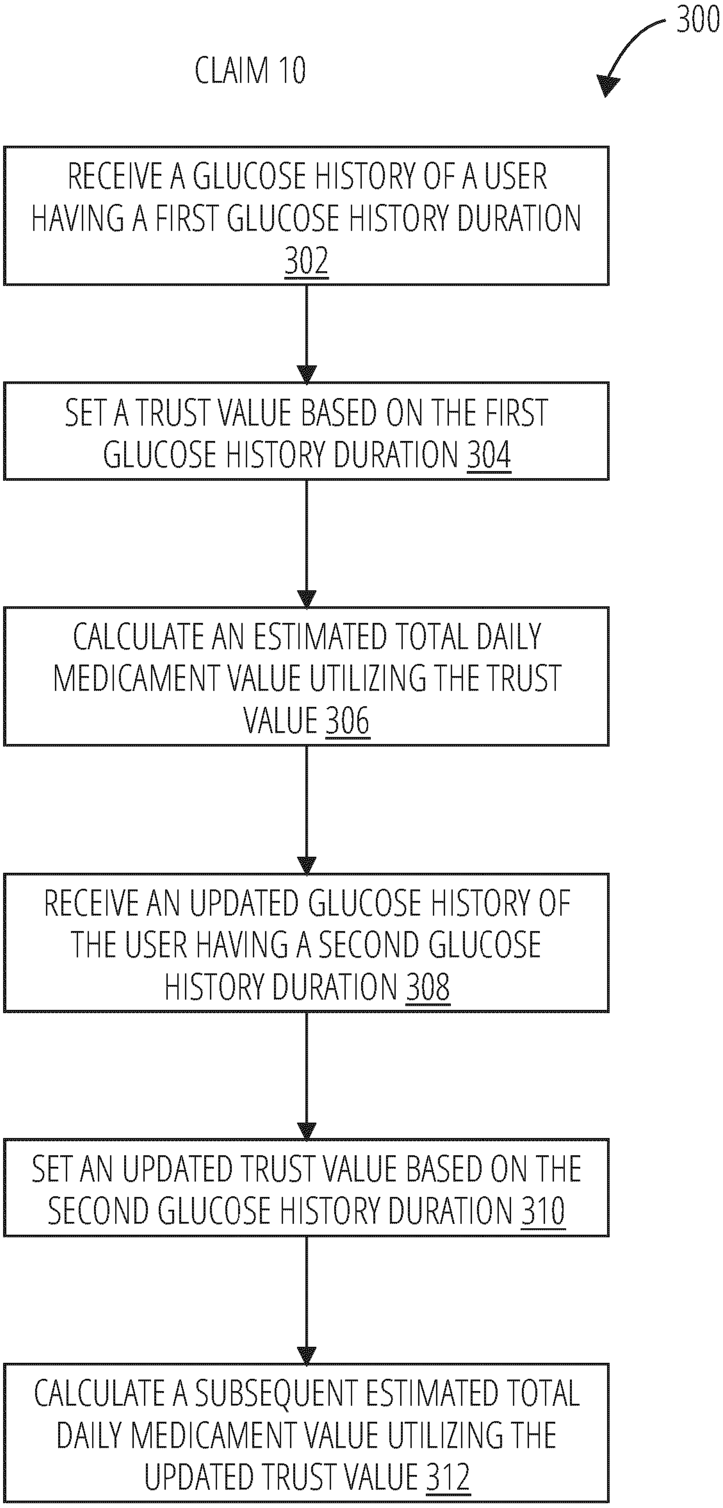
300
CLAIM 10
RECEIVE A GLUCOSE HISTORY OF A USER HAVING A FIRST GLUCOSE HISTORY DURATION 302
SET A TRUST VALUE BASED ON THE FIRST GLUCOSE HISTORY DURATION 304
CALCULATE AN ESTIMATED TOTAL DAILY MEDICAMENT VALUE UTILIZING THE TRUST VALUE 306
RECEIVE AN UPDATED GLUCOSE HISTORY OF THE USER HAVING A SECOND GLUCOSE HISTORY DURATION 308
SET AN UPDATED TRUST VALUE BASED ON THE SECOND GLUCOSE HISTORY DURATION 310
CALCULATE A SUBSEQUENT ESTIMATED TOTAL DAILY MEDICAMENT VALUE UTILIZING THE UPDATED TRUST VALUE 312

FIG. 3

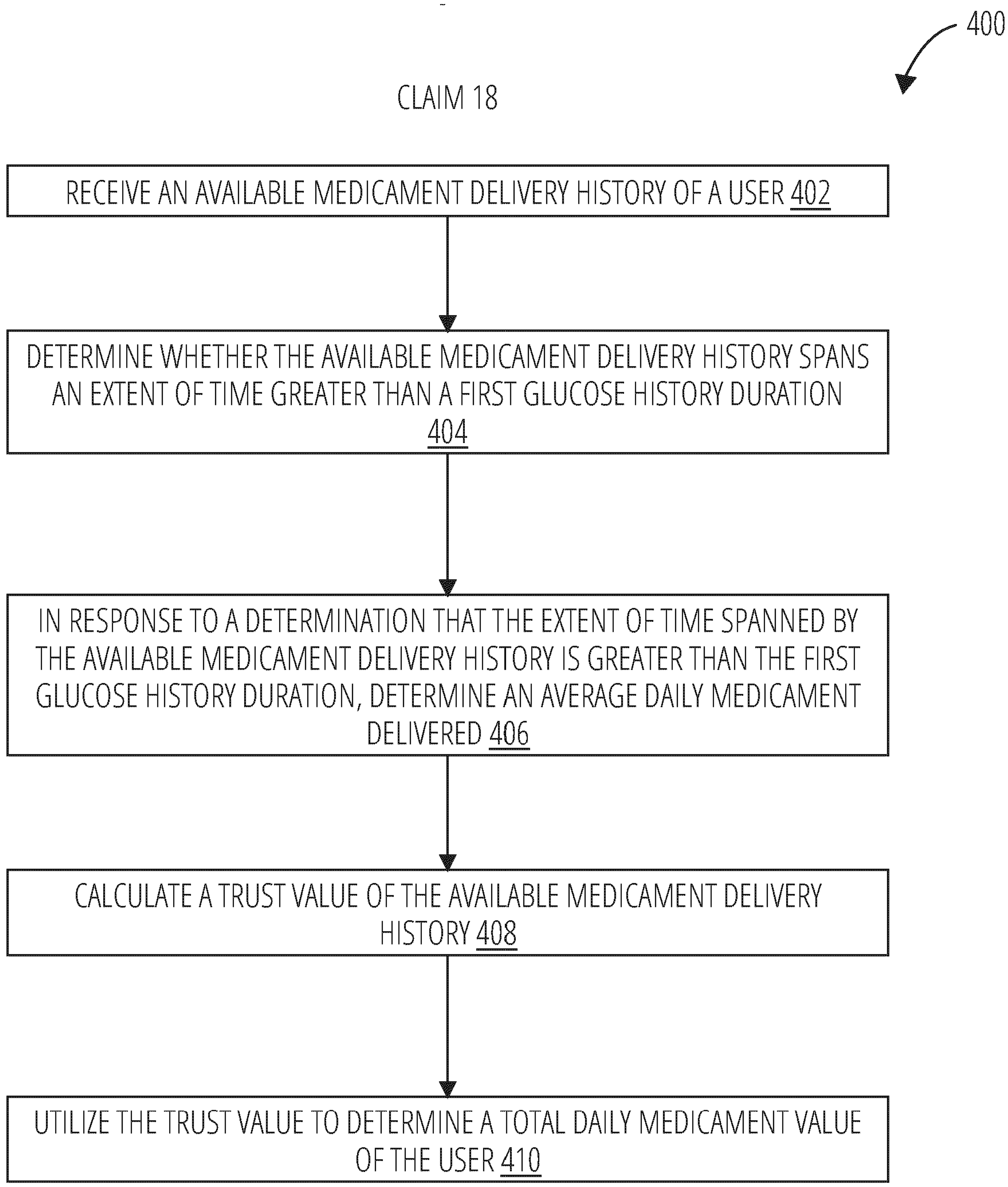
400
CLAIM 18
RECEIVE AN AVAILABLE MEDICAMENT DELIVERY HISTORY OF A USER 402
DETERMINE WHETHER THE AVAILABLE MEDICAMENT DELIVERY HISTORY SPANS AN EXTENT OF TIME GREATER THAN A FIRST GLUCOSE HISTORY DURATION 404
IN RESPONSE TO A DETERMINATION THAT THE EXTENT OF TIME SPANNED BY THE AVAILABLE MEDICAMENT DELIVERY HISTORY IS GREATER THAN THE FIRST GLUCOSE HISTORY DURATION, DETERMINE AN AVERAGE DAILY MEDICAMENT DELIVERED 406
CALCULATE A TRUST VALUE OF THE AVAILABLE MEDICAMENT DELIVERY HISTORY 408
UTILIZE THE TRUST VALUE TO DETERMINE A TOTAL DAILY MEDICAMENT VALUE OF THE USER 410

**FIG. 4**

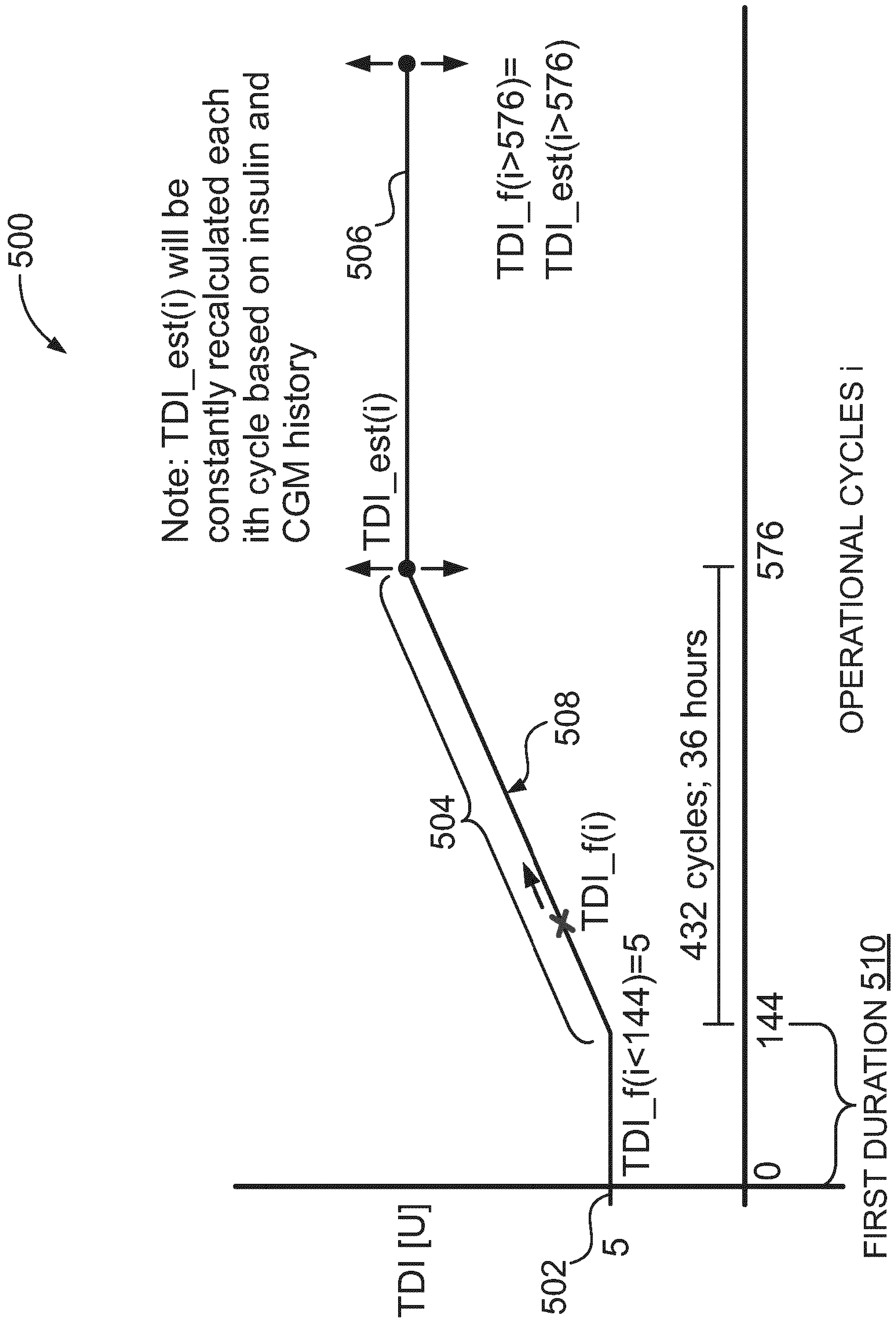
500
Note: TDI_est(i) will be constantly recalculated each ith cycle based on insulin and CGM history
TDI [U]
502
5
TDI_f(i<144)=5
504
TDI_f(i)
508
TDI_est(i)
506
TDI_f(i>576)=
TDI_est(i>576)
432 cycles; 36 hours
0
144
576
FIRST DURATION 510
OPERATIONAL CYCLES i

FIG. 5

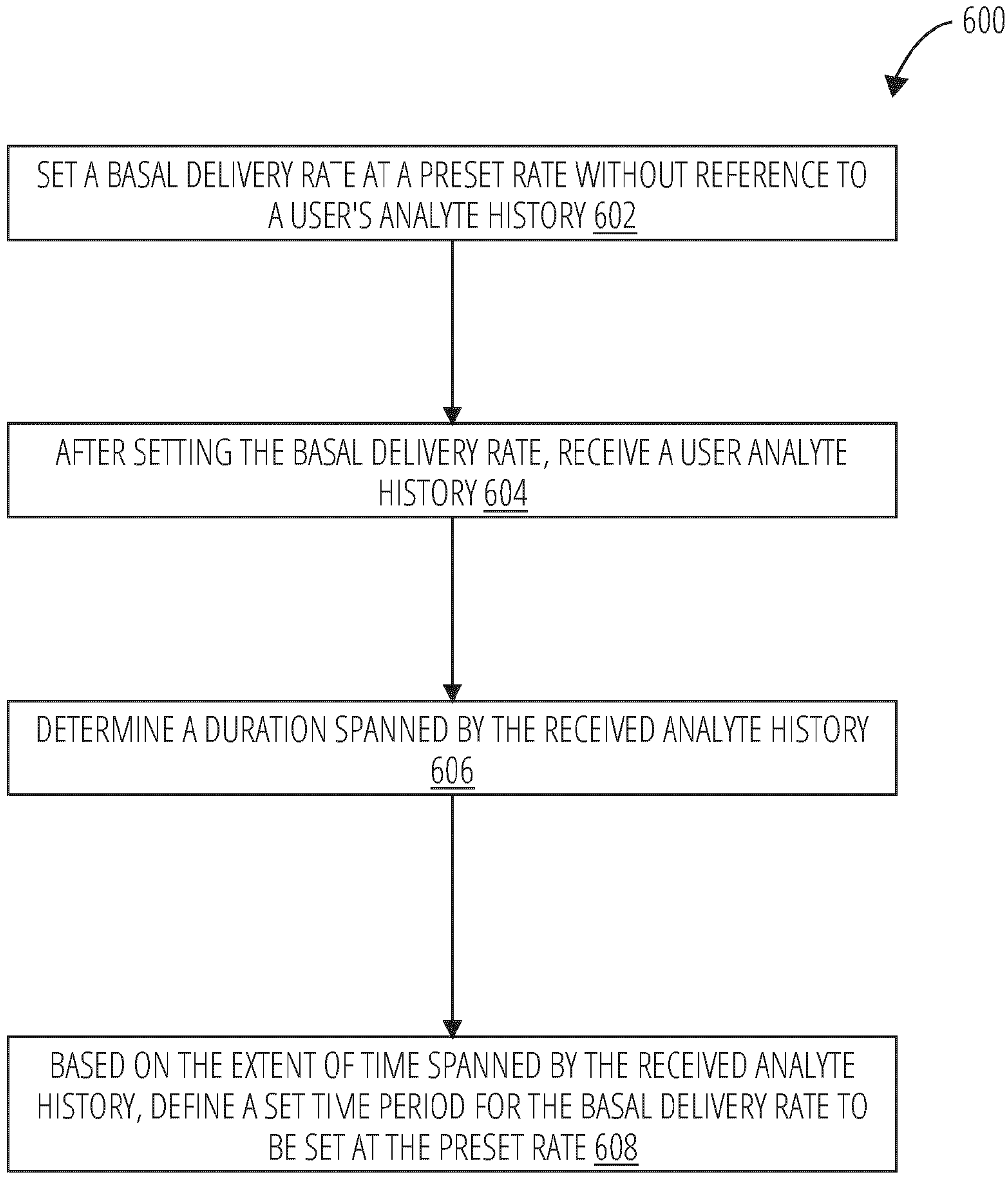
600
SET A BASAL DELIVERY RATE AT A PRESET RATE WITHOUT REFERENCE TO A USER'S ANALYTE HISTORY 602
AFTER SETTING THE BASAL DELIVERY RATE, RECEIVE A USER ANALYTE HISTORY 604
DETERMINE A DURATION SPANNED BY THE RECEIVED ANALYTE HISTORY 606
BASED ON THE EXTENT OF TIME SPANNED BY THE RECEIVED ANALYTE HISTORY, DEFINE A SET TIME PERIOD FOR THE BASAL DELIVERY RATE TO BE SET AT THE PRESET RATE 608

FIG. 6